# EUROPEAN PATENT APPLICATION

(11) **EP 1 818 328 A1**
(43) Date of publication of application: **15.08.2007**
(21) Application number: 06250732.2
(22) Date of filing: 10.02.2006
(51) Int. Cl.: C07D 305/14, A61K 31/337, A61P 35/00

(54) **Chromatographic method for the isolation and purification of taxane derivatives**

(71) Applicant: 6570763 Canada Inc., Montréal, QC H3A 3J6 (CA)
(72) Inventor: Liu, Jian, Fredericton, New Brunswick E3B 7B8 (CA)
(74) Representative: Deans, Michael John Percy

(57) **Abstract**

A method is disclosed for obtaining an organic solution of paclitaxel by way of novel purification technology. The method uses a "Load and Lock Axial Compression Column" for industrial scale preparative high performance liquid chromatography. The "Load and Lock Axial Compression Column" is one in which a piston is used to pack and unpack the chromatography bed substantially to avoid voids therein, and to maintain bed compression during use. This maintenance of the compression effectively and substantially prevents the formation of voids in the bed, which increases the efficiency of the procedure. The column can be packed with any packing material, including small particle size (e.g., about 10 µm) media. In this way, very high plate numbers are generated. The bed length can be adjusted by controlling the amount of packing material used to prepare the column. Additional steps enable paclitaxel to be isolated with a purity of 99% or more, and for crystalline 9-dihydro-1,3-acetylbaccatin III to be isolated for use in medicaments.

## Description

This invention relates to the isolation and purification of taxane derivatives from a naturally occurring *Taxus* species.

Taxol was first isolated in 1971 from the western yew, *Taxus brevifolia* by Wani, et al. Taxol is a member of the taxane family of diterpenes. Taxanes are diterpene compounds which find utility in the pharmaceutical field. Taxanes may be found in plant materials, and having been isolated therefrom. Paclitaxel is a well known chemotherapeutic drug for treatment of various metastatic cancers. It has been approved for the treatment of ovarian and breast cancers.

Taxol and various taxane derivatives are highly cytotoxic and possess strong *in vivo* activities in a number of leukemic and tumor systems. Taxol is considered to be an exceptionally promising cancer chemotherapeutic agent.

The only currently available source for taxol is several species of very slow growing yew (genus *Taxus,* family *Taxaceae).* Paclitaxel is a natural product, primarily extracted from the bark of the Pacific yew tree, *Taxus brevifolia,* and is also found in *T. baccata, T. walichiana, T. yunnanensis* and *T. canadensis.* The isolation procedures currently practised are very difficult and low-yielding. The extraction of taxol from trees of the *Taxus* genus using liquid methanol has been reported. However, taxanes are generally present in plant materials in relatively small amounts so that, in the case of taxol, for example, large numbers of the slow-growing yew trees forming a source for the compound may be destroyed. Further, large amounts of organic solvents may be employed in a conventional liquid extraction, which may be time consuming as well.

The concentration of paclitaxel in various raw materials is typically low, for example, on the order of between about 0.0004 and about 0.01 % (w/w) in the bark of Pacific yew. Such low concentrations render the extraction and purification of the compound to pharmaceutical grade from raw materials very challenging, and heretofore impractical on a commercial scale. Various normal phase chromatography techniques have been developed to purify paclitaxel from a crude extract of raw material.

The success of low pressure chromatography greatly depends on the nature of the column. Various problems are associated with the use of silica gel and alumina trioxide, all of which are classical supports of the stationary phase in partition system. They form a stable stationary phase with most solvent systems, but is a strong absorbent and may participate in the separation process to the extent that chromatographic behaviour and recovery of samples are affected.

Chromatography methods have been developed to detect and isolate paclitaxel from various *Taxus* species on analytical and preparative basis. These isolation processes are mainly conducted on a small laboratory scale and suffer from low selectivity, recovery and high production cost, thereby presenting a serious and unfulfilled need for an economically practicable method for separating the valuable anti-tumor compound paclitaxel from its close analog cephalomanine as well as other closely related taxanes.

The use of cultivable and renewable plant parts, e.g., the leaves (needles) and twigs of *Taxus* species should be the most practical and attractive way of increasing the supply of paclitaxel. The needles of several *Taxus* species, including *Taxus* canadensis, have been investigated and found to contain paclitaxel in amounts comparable to the bark of *Taxus brevifolia.*

*Taxus canadensis* is an evergreen shrub found principally in Eastern Canada and Northeastern United States. This species is unique in its taxane content. The needles contain a major taxane, 9-dihydro-1,3-acetylbaccatin III (9-DHAB III, 4) along with paclitaxel (0.009-0.05%), 10-deacetylbaccatin III (10-DAB III, 6), baccatin III, (5), cephalomannine, (3), and other minor taxanes. The concentration of 9-DHAB III in the needles is reportedly seven to ten times the concentration of paclitaxel. It appears that 9-DHAB III may be become an important precursor to a new class of semisynthetic chemotherapeutic agents with increased water solubility.

Prior art methods disclose the use of various types of chromatographic techniques to separate paclitaxel and related taxanes, including normal phase and reverse phase chromatography on a silica gel or bonded silica gel column. The prior art methods are end up at low yield, high production cost or involved multiple steps which were difficult to scale up to large industrial scale production.

The production of taxol from ornamental yew needles, barks and roots at present is not economical due to an extremely high percentage of unwanted impurities carried forward in the extract (about 40 to 50% by weight of the dried plant material) during the extraction. This unusually high percentage of impurities in the solvent extract at the needles of ornamental yew makes it very expensive and uneconomical to purify taxol and taxanes from this source in addition to the high cost in drying the needles.

The current procedures are lengthy, costly, or are practically limited to analytical scale. Since paclitaxel occurs in low levels in needles and the needles contain large amounts of waxes, the isolation and purification of paclitaxel from needles to a clinically acceptable purify pose additional challenges. A daunting task is the separation of paclitaxel from its closely related analogue cephalomannine which occurs in the needles and bark. The two analogues have been separated by selective chemical transformation of cephalomannine in a mixture containing both cephalomannine and paclitaxel. Disadvantages associated with these procedures include additional cost from the use of expensive, sometimes toxic, reagents, additional chromatography required to separate the transformed cephalomannine from paclitaxel, the destruction of cephalomannine and sometimes paclitaxel is during the process, and additional chemical transformations which are necessary for recovery of cephalomannine.

The most relevant prior art is believed to be U.S. Patent No. 5,969,165, issued October 19, 1999, to Liu, which provided a high yield and high purity method for obtaining taxane analogues from a source containing taxanes. The method employed a polymeric resin absorbent for separating the analogues under low pressure without the use of complex and extensive separation/purification steps currently provided in the art. That method for isolating and purifying taxane analogues from a source containing taxanes included the first step of extracting a source of taxanes in an organic extractant, e.g., dichloromethane. The second step involved contacting an absorbent medium with the extractant and loading the medium in a column. The column contained an absorbent agent, e.g., aluminium oxide. The next step involved eluting, with an organic solvent mixture, e.g., a mixture of hexane and acetone, at a pressure of between about 10 and 20 psi (6.89 to 13.79 * 10³ Newtons/m²) to generate fractions containing taxane compounds. The next step involved crystallizing the fractions to provide a solid taxane compound and a mother liquor; concentrating said mother liquor. The next step involved eluting, with a polar solvent mixture and mother liquor through a polymeric resin to provide at least a second taxane compound.

Thus, the art has continued to search for ever more efficient and environmentally safe methods for obtaining taxanes which minimize the use of plant materials and organic solvents.

The number of publications and patents describing the isolation and purification of paclitaxel and taxanes from *Taxus* specie is increasing, but the procedures currently known for isolation paclitaxel are very complex and difficult with a low yield, ranging from 0.005-0.017%. A list of typical such patents, below, describe various isolation technology, from normal phase chromatography to reverse phase chromatography.

U.S. Patent No.5,019,504, issued 1991, to Christen;

U.S. Patent No. 5,380,916, issued January 10, 1995, to Rao;

U.S. Patent No. 5,380,916, issued January 10, 1998 to Rao;

U S. Patent No 5,407,674, issued April 18, 1995, to Gabetta, et al.;

U.S. Patent No. 5,620,875, issued April 15, 1997, to Hoffman et al.;

U.S. Patent No. 5,670,673, issued September 23, 1997, to Rao;

U.S. Patent No. 6,503,396, issued January 7, 2003, to Kim, et al.;

Canadian Patent No. 2,126,698, issued November 8, 1993, in the name of Nair;

Canadian Patent No. 2,157,905, issued March 18, 1994, in the name of Durand et al; and

Canadian Patent 2,213,952, issued June 15, 1999, in the name of G. Caron.

As will become clear from the description below, examples of the methods disclosed herein enable the isolation of commercially important natural products from readily available biomass, an efficient method for obtaining taxanes from mixtures with other compounds or materials, an isolation procedure for paclitaxel and other taxanes from bark, needles, or cell culture of *Taxus* species which is amenable to industrial scale production, the isolation and separation of taxol and other taxanes from plant materials, preferably fresh material from ornamental yew, in high yields, a method which significantly reduces the cost of production of the taxane derivatives, and a method which provides commercial quantities of the above-referenced natural products from readily-available, renewable sources.

The present disclosure broadly teaches improvements in methods for isolating and purifying taxane analogues from a source containing the taxane analogues including the step of passing a solution containing the taxane derivatives through a chromatographic column. The improvement is characterized in that the packing media in the column has been compressed substantially to avoid voids therein, and is maintained at high pressure of e.g., about 250 to 400 psi (1.72 to 2.76 * 10⁶ Newtons/m²) or even as high as about 5000 psi (3.45 * 10⁷ Newtons/m²). The packing media in the column thus has been compressed substantially to avoid voids therein. This provides an organic solution containing impure taxane derivatives. The column is then maintained under a pressure of up to about 5000 psi. (3.45 * 10⁷ Newtons/m²), and the optional additional step of passing the organic solution containing the impure taxane derivatives through a similar chromatographic column in which a packing media in the column has been compressed to obviate voids in the column, which is then maintained at a pressure of up to about 400 psi. (2.76 * 10⁶ Newtons/m²) is carried out. The additional optional steps of passing that organic solution containing extracted said impure taxane derivatives through a similar chromatographic column in which a packing media in the column has been compressed to obviate voids in the column, which is then maintained at a pressure of up to about 400 psi (2.76 * 10⁶ Newtons/m²), to provides syrup of an extracted paclitaxel in an organic solvent are then carried out. Such syrup of the extracted paclitaxel is then passed through a similar chromatographic column in which a packing medium is compressed to obviate voids in the column, and which is maintained at a pressure of up to about 50 psi (3.45 * 10⁵ Newtons/m²). The optional preliminary steps of extracting a biomass of *Taxus* with an organic solvent, partitioning that solution between hexane and water, then repartitioning the partitioned solution between dichloromethane or chloroform and water and finally recovering a dichloromethane or chloroform solution of crude paclitaxel are carried out.

Features of the first improvement are characterized by: the selection of the organic solution as methanol, ethanol, a mixture of methanol and ethanol, a mixture of dichloromethane and methanol or a mixture of dichloromethane and ethanol; or by the selection of the packing media as diphenyl bonded silica gel or C-18 bonded silica gel; or by the elution of the column with a gradient solution of acetone/water, methanol/water or acetonitrile/water; or by the selection of the flow rate to be about 350 ml/minute, and the selection of the pressure to be maintained at about 600 to 800 psi (4.14 to 5.52 * 10⁶ Newtons/m²); or by including the step of crystallizing and recovering substantially-pure paclitaxel.

The present disclosure teaches a second improvement which is characterized by the preliminary step of passing an organic solution containing impure taxanes through a chromatographic column in which the packing media in the column has been compressed substantially to avoid voids therein, and is maintained at a high pressure of, e.g,, about 300 to 400 psi (2.07 to 2.76 * 10⁶ Newtons/m²), or even as high as 1000 psi (6.89 * 10⁶ Newtons/m²).

Features of the second improvement are characterized by: the selection of the organic solution as acetone/water, ethanol/water or isopropanol/water; or by the selection of the packing medium in the column as a polystyrene-DVB resin, or a polymethacrylate resin or a polyaromatic resin; or by the elution of the column with gradient solvent of acetone/water, methanol/water or acetonitrile/water, e.g., at a flow rate of about 1 L/minute; or by including the step of crystallizing impure paclitaxel from the eluate of the column.

The present disclosure teaches a third improvement which is characterized by passing a syrup of extracted paclitaxel in an organic solvent through a chromatographic column in which a packing medium has been compressed substantially to avoid voids therein and is maintained at a pressure of up to about 1000 psi (6.89 * 10⁶ Newtons/m²), e.g., about 30-50 psi (2.07 to 3.45 * 10⁵ Newtons/m²).

Features of the third improvement are characterized by: the selection of the packing medium as polystyrene-DVB resin, or a polymethacrylate resin or a polyaromatic resin; or by the elution of the column is with step gradients of acetone/water, methanol/water or acetonitrile/water; or by the selection of the flow rate of eluent through the column as about 350 ml/min and at a pressure of about 600 to 800 psi (4.14 to 5.52 * 10⁶ Newtons/m²), or even as high as 1000 psi. (6.89 * 10⁶ Newtons/m²); or by including the additional step of crystallizing 9-dihydro-1,3-acetylbaccatin III from a portion of the eluent from the column.

The present disclosure teaches a fourth improvement which is characterized by extracting a biomass of *Taxus* with an organic solvent and partitioning the solution between hexane and water, then re-partitioning the solution between dichloromethane or chloroform and water, and recovering a dichloromethane or chloroform solution of impure paclitaxel.

A feature of the fourth improvement is characterized by the selection of the solution of taxane analogues as a solution of crude crystalline paclitaxel in ethanol.

Thea purification technology to extract an organic solution of paclitaxel may use a "Load and Lock Axial Compression Column" for industrial scale preparative high performance liquid chromatography.

The successful use of a medium and large diameter column in preparative high performance liquid chromatography requires appropriate hardware. The "Load and Lock Axial Compression Column" is one in which a piston is used to pack and unpack the chromatography bed and to maintain bed compression during use. This effectively substantially prevents the formation of voids in the bed. The column can be packed with any packing material, including small particle size (about 10 µm) media, and very high plate numbers are generated. The bed length can be adjusted by controlling the amount of packing material used to prepare the column.

Through the use of such "Load and Lock Axial Compression Column", a commercially-economical procedure is provided for the extraction of paclitaxel from *Taxus* sp. The procedure involves a sequence of procedural steps. The last essential step may be carried out on an impure paclitaxel solution which may or may not have been prepared by the third step. Similarly, the third step may be carried out on an impure paclitaxel solution which may or may not have been prepared by the second step. Additionally, the second step may be carried out on an impure paclitaxel solution which may or may not have been prepared by the first step.

In a preferred embodiment, the first step involves the extraction from the plant material, *Taxus* sp, preferably *Taxus canadensis,* (Canadian Yew). Any part of the plant containing one or more taxanes may be employed, e.g., the bark, roots, leaves or needles, branches, twigs, seeds or whole seedlings. Preferably clipping of needles and twigs, should be dried and ground. Grinding of the plant material may be achieved by conventional means, e.g., through use of a chipper and/or a grinding mill. The taxanes can be extracted from the whole plant or from separated parts, e.g., steps, roots, leaves (needles), seeds, or mixtures thereof The material to be extracted can be either fresh or dried. Preferably, the needles are used.

The plant material is preferably first dried and ground to a suitable particle size usually ranging from about 0.001 to about 10 mm³. The plant material is extracted using an organic solvent, e.g., methanol, ethanol, a mixture of methanol and ethanol, a mixture of dichloromethane and methanol, or a mixture of dichloromethane and ethanol. The filtrate from the extractant is mixed with water and is concentrated in order to reduce the volume of the liquid. The concentrate is partitioned between hexane and water in order to effect the step of defatting. The aqueous liquor is repartitioned between an organic solvent, e.g., dichloromethane, chloroform or ethyl acetate and water, and is concentrated to a thick syrup.

In a preferred embodiment, the second step involves passing the thick syrup through a column. The preferred solution for this ion exchange is the thick syrup prepared in the first step, which is diluted with an organic solvent, e.g., acetone, ethanol or isopropanol. However, any suitable organic solution containing extracted paclitaxel may be the starting material in this second step.

The syrup in, e.g., dichloromethane/acetone/water, is separated into several fractions by pouring through a medium pressure column containing a polymeric absorbent resin. The polymeric absorbent resin may be, e.g., a polystyrene DVB resin, a polymethacrylate resin or any other suitable polyaromatic resin. The most common type of resin used in polymeric absorbent resins are polystyrene polymers with crosslinking divinylbenzenes. The polymeric absorbent resin should have a suitable particle size, which is thus suitable for the column size and the designated working pressure and flow rate. One non-limiting example is a particle size of about 5 to 100 mesh.

The eluent solution is passed through the column with step gradients of a suitable water-miscible solvent, e.g., acetone/water, methanol/water, ethanol/water or acetonitrile/water. The flow rate is variable and can be changed according to the various operating conditions. One non-limiting example is a flow rate of about 2L/min.

The fractions containing up to about 45% acetone/water are allowed to form crystals of 9-dihydro-1,3-acetylbaccatin III.

The fractions containing more than about 45% acetone/water are concentrated to remove most of the acetone and are extracted with a suitable organic solvent, e.g., ethyl acetate, CH₃CL or CH₂Cl₂, and is concentrated to provide a crude solid residue of paclitaxel.

The third step involves the purification of crude solid paclitaxel. Preferably, this crude solid paclitaxel is the product of the second step. However, crude solid paclitaxel however produced may be the starting material for this third step.

The crude solid paclitaxel is dissolved in a suitable water-miscible solvent, e.g., acetone, in order to affect the step of defatting, and the water-miscible organic solvent/water solution, e.g., an acetone/water solution thereof, is eluted by way of axis compression column chromatography. This system is a high performance liquid chromatography system, with means to provide and maintain a high pressure, e.g., about 250 to 400 psi (1.72 to 2.76 * 10⁶ Newtons/m²) or even as high as about 5000 psi (3.45 * 10⁷ Newtons/m²).

This column containing a suitable ion exchange resin as described above is loaded with the paclitaxel solution and is eluted with gradients of, e.g., acetone/water, at the above high pressure. The fractions containing paclitaxel in such acetone/water were allowed to crystallize, and dried to provide semi-pure paclitaxel.

The final step involves the purification of the semi-pure paclitaxel. Preferably, the semi-pure paclitaxel is the product of the third step. However, semi-pure paclitaxel, however produced, may be the starting material for the final step.

The column used in the final step is the same column as used in the third step. The ion exchange resin, or packing medium in the column may be diphenyl bonded silica gel or C-18 bonded silica gel or any other reverse phase packing material.

The semi-pure paclitaxel is dissolved in a suitable water-miscible solvent, e.g., acetonitrile, ethanol or methanol. This solution is loaded into the packed column and is eluted with gradient solvents of the water-miscible organic solvent/water solution, e.g., acetonitrile/water, at a suitable rate, e.g., about 350 ml/min and a suitable pressure maintained at, e.g., about 600 to 800 psi (4.14 to 5.52 * 10⁶ Newtons/m²), or even as high as about 1000 psi. (6.89 * 10⁶ Newtons/m²).

The fraction containing pure paclitaxel was allowed to crystallize, and the crystals were then recrystallized from acetone/hexane. A pure white powder paclitaxel is provided.

A number of non-limiting examples are set out below.

### Example 1:

### Extraction of Biomass:

The plant material (Canadian yew, *Taxus canadensis)* was harvested in the Canadian provinces of P.E.I. and New Brunswick in the fall 2002, and contained about 0.032% of paclitaxel. The fresh clippings of the Canadian yew were dried in a drying kiln at about 60 to 70°C before grinding.

600 kilograms of the ground needles and twigs of biomass was placed into a 3000 extractor which equipped with a reflecting condenser. 2400 L of methanol were then added. The biomass was refluxed with methanol for about 3 hours, the solvent was filtered, and the first filtrate was collected. 2000 L of methanol was then added to the biomass reside after filtration, was reflexed for about 2 hours, and was then filtered. The second filtrate was collected and the biomass was discharged. The first and second filtrates were combined and about 10% water was added. The aqueous solution was concentrated in an evaporator under vacuum to about 15% of its original volume.

The concentrate was twice partitioned between hexane and water to defat the concentrate. The aqueous layer was collected and was twice re-partitioned between dichloromethane (or chloroform) and water. The organic layer was collected and concentrated in an evaporator under vacuum to a thick syrup. The hexane layer was recovered, and the water layer was discharged.

Approximately 45 kilograms of syrup was obtained from 600 kilograms of biomass. The dichloromethane syrup contained approximately 1 to 1.3% of paclitaxel.

### Example 2:

### Primary Purification of Paclitaxel:

23 Kilograms of the dichloromethane syrup from Example 1 were diluted with 10 L of acetone in a container equipped with a mechanical stirrer, with warming if necessary. The stirring was adjusted to 1.5 rotations per second and 3 L of deioned water was added gradually over a period of 10 minutes. The aqueous solution of the dichloromethane syrup was separated into several fractions by a stainless steel, industrial scale, medium pressure column which was equipped with a medium pressure metering diaphragm pump.

A slurry of 120 kilograms of polystyrene-DVB resin (Rohm-Hass XAD-1600), with a particle size of about 50 to 100 mesh, in methanol, was poured into the column which was 300 mm diam. and 3000 mm long. After the column had settled while the solvent was pumped at about 30 to 50 psi (2.07 to 3.45 * 10⁵ Newtons/m²), the methanol was replaced with about 30% acetone in water until the column was equilibrated.

The diluted syrup sample was transferred to the top of the column. The container was rinsed with a few L of 30% acetone in water and the slurry was transferred to the column. The column was then sealed and started to flow as the elution solvent was pumped into the column. The column was then eluted with step gradients of 35, 45, 50, 60, 65, 70 and 80% acetone/water. The flow speed was controlled at about 2 L per minute. Fractions of approx. 20 liter each were collected with the pressure maintained between about 30 to 50 psi (2.07 to 3.45 * 10⁵ Newtons/m²).

After first washing with 80% acetone/water, the column was washed with pure acetone, followed by washing with a mixture of acetone/ethyl acetate (1:1) until the effluent, which was initially very dark, become colorless.

After washing, the column was equilibrated again with 30% acetone in water, at which point it was regenerated and was ready for next run.

The fractions which were collected were monitored by UV absorbance at 227/nm with analytical HPLC, and with TLC monitored by spraying, 10% H₂O in ethanol. The fractions (most in 45% acetone/water) containing 9-dihydro-13-acetylbaccatin III were combined and concentrated to remove most of acetone and left in a hood overnight until crystallization completed. The crystals were filtered out and re-crystallized from methanol to yield 240 grams of pure 9-dihydro-1,3-acetylbaccatin III as white, needle like crystals. Yield: 0.08%.

The fractions (most in 65% acetone/water) containing paclitaxel were combined and concentrated to remove most of acetone. The aqueous solution was extracted with ethyl acetate. The organic phase was collected and concentrated to dryness. The residue was analyzed by analytical HPLC. The residue (crude paclitaxel material) contained about 10 to 15% paclitaxel. The recovery of paclitaxel was between about 90 to 100% from the dichloromethane syrup.

### Example 3:

### Secondary Purification of Paclitaxel:

The secondary purification used a preparative high performance liquid chromatography system, which is called "axis compression column chromatography". The column used was a Varian Load and Lock axis compression column (250 mm diam. X 1000 mm long). The chromatographic system was a Varian ST-4000 system, which was equipped with two high pressure diaphragm pumps, a sample inlet port, a UV detector, and a Varian control software system. The column was equipped with a hydraulic piston for packing and unpacking the column and lock adaptor. After full packing the column with 50 liter of polystyrene-DVB resin (Rohm-Hass CG-161 m, particle size about 75 µm) in ethanol, the hydraulic piston was turned on. The piston slowly moves out to compress the column packing media at a pressure of about 3000 psi (2.07 * 10⁷ Newtons/m²). While the column was compressed, the lock was switched to its locking position to lock the column. Thus, the column can generate a very high plate number. After the column had settled while the solvent was pumped at about 250 to 300 psi (1.72 to 2.07 * 10⁶ Newtons/m²), the ethanol was replaced with about 30% acetone in water until the column was equilibrated.

The residue from Example 2, approx. 1.5 kilograms, was dissolved in 4 L of acetone until all solid was dissolved. 2 L of water was gradually added with stirring, and was filtered if necessary. The aqueous solution was then pumped into the column though the sample inlet port.

The column was then eluted with gradient solvents of acetone/water (starting from 50% acetone in water, ending at 100% acetone). The flow rate was controlled at 1 L per minute. Fractions of approx. 5 liter each were collected with the pressure maintained at between about 300 to 400 psi (2.07 to 2.76 * 10⁶ Newtons/m²).

The fractions collected were monitored by U.V. absorbance at 227/mm with analytical HPCL and with TLC monitored by spraying 10% H₂O in ethanol. The fractions (most in 70% acetone/water) containing paclitaxel were combined and concentrated to remove most of acetone and were left in a hood overnight until crystallization completed. The crystals were filtered out and re-crystalized from 70% methanol in water and dried in a vacuum oven to yield 235 grams of slightly white, needle-like crystals. This semi-pure paclitaxel had a purity of about 70 to 80%. Yield: about 90 to 95% from the crude paclitaxel material.

### Example 4:

### Final Purification of Paclitaxel:

The final purification used a preparative high performance liquid chromatography system, and an axis compression chromatographic column. The column used was a Varian Load and Lock axis compression high pressure column (100 mm diam. X 1000 mm long). The chromatographic system was a Varian SD-2 preparative HPLC system, which was equipped with a sample inlet port, a UV detector, and a Varian control software. The column was equipped with a hydraulic piston for packing and unpacking the column and with a lock adaptor. The packing media used was diphenyl bonded silica gel or C-18 bonded silica gel.

After full packing the column with 6 liter of packing media in ethanol, the hydraulic piston was turned on. The piston slowly moved out to compress the column packing media at a pressure of about 3000 psi (2.07 * 10⁷ Newtons/m²). While the column was compressed, the Lock was switched to its locking position to lock the column. Before using the column for purification, the ethanol was replaced with about 30% acetonitrile in water until the column was equilibrated.

The crude paclitaxel from Example 3, approx. 50 grams, was dissolved in 200 ml of acetonitrile until all solid was dissolved. 100 ml of water was gradually added with stirring and was filtered if necessary. The aqueous solution was then pumped into the column through the sample inlet port.

The column was then eluted with gradient solvents of acetonitrile/water (starting from 30% acetonitrile in water, ending at 100% acetonitrile). The flow rate was controlled at about 350 ml per minute. Fractions of approx. 1 L each were collected with the pressure maintained between about 600 to 800 psi (4.14 to 5.52 * 10⁶ Newtons/m²).

The fractions collected were monitored by U.V. detector with an absorbance at 227/nm, and was monitored with TLC by spraying about 10% H₂O in ethanol. The fractions containing pure paclitaxel were combined and concentrated to remove most of acetonitrile and left in a hood overnight until crystallization was completed. The crystals were filtered out and re-crystallized from acetone: hexane (1:1) and dried in a vacuum oven to yield 36.5 grams of white powder. This pure paclitaxel has a purity of greater than about 99%. Yield: about 85 to 90% from the about 70 to 80% pure crude paclitaxel.

The total overall yield is greater than about 73%.

The methods of which examples are described herein can provide paclitaxel and other taxanes in high yield and purity on industrial scale. The methods of which examples are described herein can provide cost-effective processes for mass production of paclitaxel and other related taxanes from a vegetal source or tissue culture, particularly from *T. canadensis.*

The methods of which examples are described herein enable mass production of paclitaxel and related taxanes from plants of the genus *Taxus* (Taxaceae).

The isolation and purification processes described herein by way of example permit a highly efficient recovery of taxane derivatives in pure form from a naturally-occurring *Taxus* species. The improvement these processes provide over the prior art is reflected in the high overall recovery yield of the taxanes as well as in the purity of the taxanes isolated.

## Claims

1. A method for isolating and purifying taxane analogues from a source containing said taxane analogues including the step of passing an organic solution containing said taxane derivatives through a chromatographic column to provide an organic solution containing impure taxane derivatives, the column having a packing medium that has been compressed substantially to avoid voids therein and is then maintained under a pressure of up to about 5000 psi (3.45 * 10⁷ Newtons/m²); optionally carrying out the additional step of passing said organic solution containing said impure taxane derivatives through a chromatographic column in which a packing medium in said column has been compressed to obviate voids in said column and is then maintained at a pressure of up to about 400 psi (2.76 * 10⁶ Newtons/m²); optionally carrying out a second additional step of passing that organic solution containing extracted said impure taxane derivatives through a chromatographic column in which a packing medium in said column has been compressed to obviate voids in said column and is then maintained at a pressure of up to about 400 psi (2.76 * 10⁶ Newtons/m²), thereby to provide an extracted paclitaxel in an organic solvent; and passing a syrup of said extracted paclitaxel in an organic solvent through a chromatographic column in which a packing medium is compressed to obviate voids in said column, and is maintained at a pressure of up to about 50 psi (3.45 * 10⁵ Newtons/m²); the method further optionally including preliminary steps of extracting a biomass of *Taxus* with an organic solvent, partitioning said solution between hexane and water, then repartitioning the solution between dichloromethane or chloroform and water, and finally recovering a dichloromethane or chloroform solution of crude paclitaxel.

2. A method according to Claim 1, wherein said solution of taxane analogues comprises a solution of crude crystalline paclitaxel in methanol, ethanol, a mixture of methanol and ethanol, a mixture of dichloromethane and methanol or a mixture of dichloromethane and ethanol.

3. A method according to Claim 1 or Claim 2, wherein said packing medium is a diphenyl-bonded silica gel; or is a C-18 bonded silica gel; or is a polystyrene-DVB resin; or is a polymethacrylate resin; or is a polyaromatic resin.

4. A method according to any preceding Claim, wherein said column is eluted with a gradient solution of acetone/water, methanol/water or acetonitrile/water.

5. A method according to any preceding Claim, wherein the flow rate is about 350 ml/minute, and the pressure is maintained at about 600-800 psi (4.14 to 5.52 * 10⁶ Newtons/m²).

6. A method according to Claim 5, wherein the flow rate is about 1 L/minute to about 2 L/minute.

7. A method according to any preceding Claim, wherein the organic solvent in said first mentioned step comprises dichloromethane, and the solution to be eluted through said column in said second optional step comprises acetone/water, methanol/water or acetonitrile/water.

8. A method according to any preceding Claim, wherein the organic solvent is dichloromethane, and the eluant solution through said column is acetone/water; or methanol/water; or ethanol/water; or isopropanol/water; or acetonitrile/water.

9. A method according to any preceding Claim, further including the additional step of crystallizing paclitaxel from the eluate of said column.

10. A method according to any one of Claims 1 to 8, further including the additional step of crystallizing 9-dihydro-1,3-acetylbaccatin III from a portion of eluent from said column.

11. Paclitaxel isolated with a purity of 99% or more.

12. Isolated crystalline 9-dihydro-1,3-acetylbaccatin III.

13. A medicament comprising Paclitaxel isolated with a purity of 99% or more or isolated crystalline 9-dihydro-1,3-acetylbaccatin III, together with pharmacologically acceptable diluents.
